(19) Europäisches Patentamt
European Patent Office
Office européen des brevets

(11) Numéro de publication: **0 409 684 A1**

# (12) DEMANDE DE BREVET EUROPEEN

(21) Numéro de dépôt: **90401898.3**

(22) Date de dépôt: **29.06.90**

(51) Int. Cl.5: **C07C 49/835**, C07C 317/22, C07C 255/53, C07C 49/813, C07C 317/14, C07C 255/50, C08G 75/23, C08G 65/48, C07C 205/57, //C07C317/32

(30) Priorité: **19.07.89 FR 8909720**

(43) Date de publication de la demande: **23.01.91 Bulletin 91/04**

(84) Etats contractants désignés: **AT BE CH DE FR GB GR IT LI LU NL**

(71) Demandeur: **INSTITUT NATIONAL DE RECHERCHE CHIMIQUE APPLIQUEE**
**18 Bis, Boulevard de la Bastille**
**F-75012 Paris(FR)**

(72) Inventeur: **Delfort, Bruno**
**15 rue Broca**
**F-75005 Paris(FR)**
Inventeur: **Lucotte, Georges**
**41 Domaine du Château**
**F-91380 Chilly Mazarin(FR)**
Inventeur: **Cormier, Laurent**
**23 rue Claude Vellefaux**
**F-75010 Paris(FR)**

(74) Mandataire: **Rinuy, Santarelli**
**14, avenue de la Grande Armée**
**F-75017 Paris(FR)**

## (54) Nouveaux dérivés acétyléniques, leur procédé de préparation, nouveaux polymères acétyléniques et leurs applications.

(57) Dérivés de formule générale

(I)

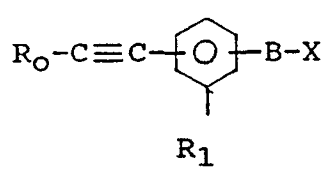

dans laquelle $R_0$ représente un atome d'hydrogène ou un radical isopropanol ou triméthylsilyl, $R_1$ représente un atome d'hydrogène ou un radical cyano ou nitro, X représente un atome de chlore ou de fluor ou un radical nitro et B représente une liaison carbone-halogène ou un radical :

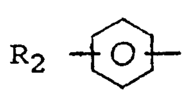

dans lequel $R_2$ représente un groupement carbonyle ou sulfone, si B = $-R_2-O-$, $R_1$ = H, si B = liaison C-Hal, $R_1$ est différent de H; procédé de préparation, polymères α-w-acétyléniques portant un groupement

EP 0 409 684 A1

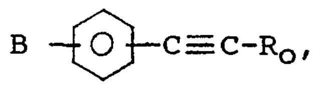

applications et produits conformés.

## NOUVEAUX DÉRIVÉS ACÉTYLÉNIQUES, LEUR PROCÉDÉ DE PRÉPARATION, NOUVEAUX POLYMÈRES ACÉTYLÉNIQUES ET LEURS APPLICATIONS.

La présente invention concerne de nouveaux dérivés acétyléniques, leur procédé de préparation, de nouveaux polymères acétyléniques et leurs applications.

On a déjà décrit dans le brevet US-4.431.761 et dans J. of Polym. Sci.-Pcl. Chim. Ed.. 22 , 3011, 1984, la préparation de polymères de type polyaryléther comportant des fonctions acétylène terminales.

Les fonctions acétylène sont reliées aux polymères de base par une liaison ester obtenue par action du chlorure de 4-éthynyl-benzoyle. Les polymères ainsi fonctionnalisés sont sensibles à l'hydrolyse et aux attaques acides et alcalines.

C'est pourquoi la présente demande concerne de nouveaux dérivés de l'acétylène permettant la fonctionnalisation de polymères ou d'oligomères hydroxylés qui, contrairement à ceux de l'art antérieur, ne sont pas sensibles à l'hydrolyse ni aux attaques acides et basiques. Les fonctions éther qui peuvent être obtenues grâce aux dérivés de l'invention sont particulièrement résistantes vis-à-vis des agents chimiques, sont stables thermiquement ainsi qu'en milieu acide et basique et ne sont pas hydrolysables. Par ailleurs, les dérivés de la présente invention permettent au cours de la même étape la synthèse de polymères et leur fonctionnalisation acétylénique.

La présente demande a donc pour objet des dérivés acétyléniques, caractérisés en ce qu'ils répondent à la formule générale (I) :

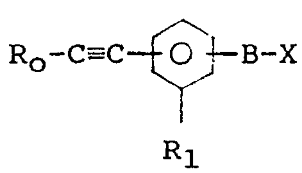

(I)

dans laquelle $R_0$ représente un atome d'hydrogène ou un radical isopropanol ou triméthylsilyl, $R_1$ représente un atome d'hydrogène ou un radical cyano ou nitro, X représente un atome de chlore ou de fluor ou un radical nitro et B représente une liaison carbone-halogène ou un radical :

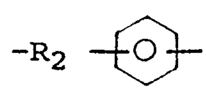

dans lequel $R_2$ représente un groupement carbonyle ou sulfone, étant entendu que si B représente un radical

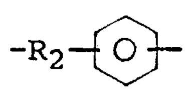

$R_1$ représente un atome d'hydrogène et si B représente une liaison carbone-halogène, $R_1$ ne représente pas un atome d'hydrogène.

Parmi les dérivés objet de l'invention, on retient de préférence les dérivés de formule (I), caractérisés en ce que $R_0$ représente un atome d'hydrogène et ceux pour lesquels B représente un radical

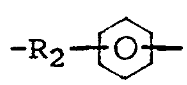

Parmi ceux-ci, on retient notamment ceux caractérisés en ce que $R_2$ représente un groupement carbonyle et X représente un atome de fluor.

La présente invention a également pour objet un procédé de préparation des dérivés ci-dessus décrits,

caractérisé en ce que l'on fait réagir un dérivé de formule (II) :

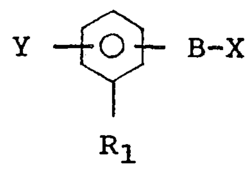

(II)

dans laquelle Y représente un atome de brome ou d'iode et B, X et $R_1$ ont la signification déjà indiquée, avec un dérivé de l'acétylène de formule (III) :

$R_0$-C≡CH (III)

dans laquelle $R_0$ a la signification déjà indiquée, à l'exception de l'hydrogène, pour obtenir un dérivé de formule (IA) :

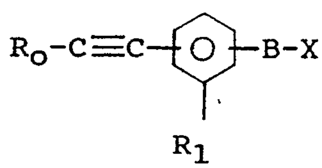

(IA)

dans laquelle $R_0$, $R_1$, B et X ont la signification déjà indiquée que soit l'on isole, soit l'on clive pour obtenir un dérivé de formule (IB) dans laquelle $R_1$, B et X ont la signification déjà indiquée :

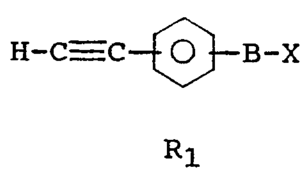

(IB)

Les produits de formule (II) sont connus ou décrits dans la littérature. Certains peuvent également être préparés par réaction de Friedel et Crafts entre un dérivé de formule

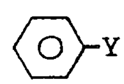

et un dérivé de formule :

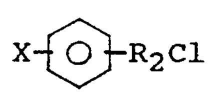

ou un dérivé de formule

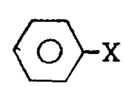

et un dérivé de formule :

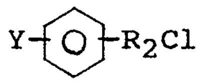

dans lesquelles X et Y ont la signification déjà indiquée.

Des exemples de telles préparations sont donnés ci-après dans la partie expérimentale.

La réaction du dérivé de formule (II) avec le dérivé acétylénique de formule (III) est réalisée de préférence en présence d'une base, notamment une amine tertiaire telle que la pyridine ou une trialkylamine, de préférence la triéthylamine.

De telles bases servent avantageusement elles-mêmes de solvants réactionnels. Il est cependant possible d'ajouter un autre solvant.

Pour la mise en oeuvre de cette réaction, l'on peut également utiliser comme milieu réactionnel une base solide dispersée dans un solvant. On utilise alors par exemple un carbonate ou un hydrogénocarbonate alcalin, de préférence de sodium ou de potassium dans un solvant tel que le toluène, le chlorobenzène, le dichlorobenzène, le nitrobenzène ou la N-méthylpyrrolidone.

Le substituant Y du produit de départ de formule (II) est avantageusement un atome d'iode.

La réaction du dérivé de formule (II) avec le dérivé de formule (III) est avantageusement réalisée en présence d'un catalyseur à base de palladium. On utilise par exemple le dichloropalladium ou le diacétate de palladium mais de préférence le système catalytique à base de bis-(triphénylphosphine)-dichloropalladium, de triphénylphosphine et d'iodure de cuivre.

Le clivage du dérivé de formule (IA) est avantageusement réalisé à l'aide d'une base telle que l'hydrure de sodium, d'un carbonate ou d'un hydrogénocarbonate alcalin, de préférence de potassium, et notamment à l'aide d'un hydroxyde alcalin, particulièrement de sodium ou de potassium.

Les dérivés de formule (I) présentent de remarquables qualités qui les rendent particulièrement intéressants pour l'élaboration d'oligomères ou de polymères comportant des liaisons acétyléniques réactives en bout de chaîne. En effet, les groupements acétyléniques peuvent s'associer ou se polymériser par réaction d'addition. De plus, l'halogène des dérivés de formule (I) est facilement éthérifiable par des fonctions alcool aliphatiques ou phénol.

Parmi les dérivés pouvant être fonctionnalisés à l'aide des dérivés de formule (I), on peut citer notamment les oligomères ou polymères porteurs de groupements hydroxyle en bout de chaîne et notamment les polyaryléthers tels que les polysulfones, les polyéthersulfones, les polyéthercétones terminées par des fonctions phénols, les polyesters.

Ces oligomères ou polymères fonctionnalisés par les dérivés de formule (I) comportent donc à leurs extrémités des groupements acétyléniques ; ils seront eux-mêmes précurseurs de polymères obtenus par association ou polymérisation desdites fonctions acétyléniques sous l'action, par exemple, de la chaleur.

C'est pourquoi la présente demande a également pour objet les polymères $\alpha$-w-acétyléniques, caractérisés en ce que la fonction acétylénique est portée par un groupement :

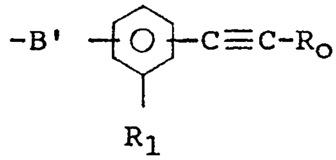

terminal dans lequel $R_0$ et $R_1$ ont la signification déjà indiquée et $B'$ représente une liaison C-O ou un radical-

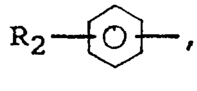

relié auxdits polymères par une fonction éther, $R_2$ ayant la signification déjà indiquée.

Les polymères ci-dessus sont avantageusement des polyaryléthers.

Selon une caractéristique importante de l'invention, la préparation des polymères ou oligomères ci-dessus, notamment les polyaryléthers terminés par des chaînes acétyléniques pourra être effectuée en une étape unique par réaction par exemple d'un excès de bisphénol sur un dihalogénure aromatique en

présence de dérivés de formule (I) selon l'invention.

Cette réaction pourra être réalisée notamment en milieu basique, par exemple en présence de soude, de potasse, de carbonate ou d'hydrogénocarbonate de potassium ou de sodium, dans un solvant généralement polaire tel que le diméthylacétamide, le diméthylsulfoxyde ou la N-méthylpyrrolidone.

Une telle réaction peut être schématisée comme suit : on fait réagir le bisphénol de formule (IV) :

$HO-R_3-OH$ (IV)

dans laquelle $R_3$ représente :

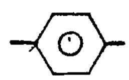

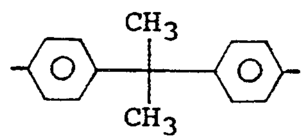

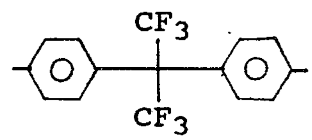

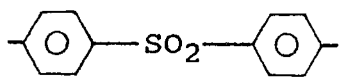

ou

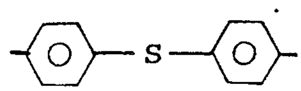

en excès sur un dihalogénure aromatique de formule (V) :

$X'-R_4-X'$ (V)

dans laquelle $R_4$ représente :

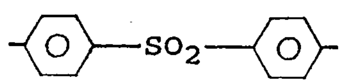

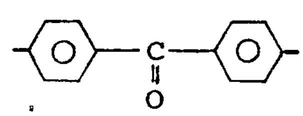

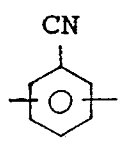

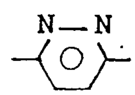

ou

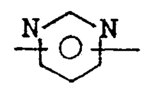

et $X'$ représente un atome de fluor ou de chlore, en présence d'un dérivé de formule (I) pour obtenir les dérivés de formule (VI) attendus :

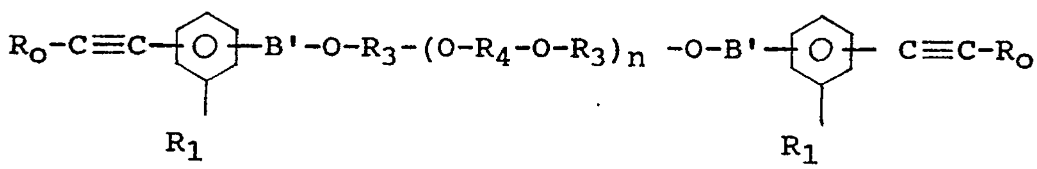

dans laquelle $B'$, $R_0$, $R_1$, $R_3$ et $R_4$ ont la signification déjà indiquée.

Des exemples de telles préparations ainsi que les mises en oeuvre du produit obtenu sont illustrés ci-après dans la partie expérimentale.

La présente demande a également pour objet l'application des dérivés ci-dessus à l'éthynilation de dérivés polymériques ou oligomériques hydroxylés.

Dans le cas de polyesters, on opèrera en deux étapes, la première consistant à préparer des polyesters α-w-hydroxylés par exemple en faisant réagir un excès de diol tel que le polypropylène ou l'éthylèneglycol ou l'hexane-diol, ou un diphénol tel que le bisphénol A avec un diacide aliphatique ou aromatique tel que l'acide iso- ou téréphtalique, ou encore l'acide adipique, et la seconde consistant à faire réagir ce dipolymère avec des composés de formule (I) dans les conditions décrites ci-dessus.

Les polymères acétyléniques ci-dessus sont dotés notamment d'une température de fusion relativement basse, généralement inférieure à 200 °C lorsque $R_0$ représente l'hydrogène et présentent une certaine solubilité dans les solvants. Ces polymères peuvent être mis en forme classiquement par fusion, moulage, extrusion, injection, compression. De plus, leur solubilité leur confère de bonnes qualités d'imprégnation,

par exemple pour la réalisation de composites. La mise en oeuvre de ces polymères ou oligomères fonctionnalisés par les dérivés de formule (I) permet d'obtenir des matériaux généralement réticulés présentant une tenue aux solvants et des propriétés thermiques et mécaniques améliorées par rapport à leurs précurseurs.

Ces produits sont dotés à la fois des propriétés des polymères thermoplastiques, telles que la ténacité, c'est-à-dire l'absence de fragilité, propagation très limitée des fissures -propriétés mises en évidence par le test dit de K1C- et celles des polymères thermodurcissables telles que la tenue aux solvants.

Pour des mises en oeuvre simples de quelques minutes à des températures comprises entre 180 et 270° C, les matériaux obtenus grâce aux polymères fonctionnalisés selon l'invention présentent des températures de transition vitreuse comprises entre 170 et 210° C, des facteurs d'intensité de contrainte critique (K1C) compris entre 0,8 et 3,9 $MPa.m^{0,5}$ et une bonne tenue aux solvants tels que le chloroforme.

C'est pourquoi la présente demande a aussi pour objet les objets conformés caractérisés en ce qu'ils comprennent un polymère ou oligomère tel que défini ci-dessus.

La mesure de la thermostabilité de ces produits montre que leur température de début de perte de poids est de l'ordre de 405 à 470° C.

Ces polymères traités sont particulièrement adaptés à leur utilisation comme matrice de matériaux composites.

Des exemples de telles mises en oeuvre figurent ci-après dans la partie expérimentale.

En plus des propriétés déjà indiquées ci-dessus, ils permettent la synthèse de polyéthers et leur fonctionnalisation terminale acétylénique au cours de la même étape réactionnelle, ce qui les rend particulièrement attractifs vis-à-vis de ceux de l'art antérieur qui nécessitaient d'une part l'obtention d'un polymère avec des terminaisons, notamment phénoliques, isolé et sec et, d'autre part, de procéder ensuite à leur fonctionnalisation par estérification.

De plus, les dérivés selon la présente invention sont préparés et utilisés avec de remarquables rendements.

Les exemples qui suivent illustrent la présente invention sans toutefois la limiter.

Exemples 1 à 7

Greffage d'un groupe acétylénique

On ajoute en présence d'une base 1,075 mole d'halogénure de formule (II) en solution à 0,16 mole de 3-méthyl-3-ol-butyne-1 puis 107 mg de Pd $(\phi_3P)_2Cl_2$, 215 mg de $P\phi_3$ et 215 mg de CuI. On porte le mélange à 90° C, durant 7 heures. A l'issue de ce temps, on filtre le milieu, lave avec une solution aqueuse d'acide chlorhydrique puis avec de l'eau et évapore le solvant pour obtenir le produit désiré.

Les détails des synthèses et les caractéristiques analytiques figurent au tableau I.

Exemples 8 à 14

Déprotection de la fonction acétylénique

On disperse 2,5 g de soude finement divisée dans une solution de 17 mmoles de dérivé acétylénique bloqué préparé précédemment dans 150 $cm_3$ de chlorobenzène. On porte alors à 110° C pendant 2 heures. On filtre, lave avec deux fois 200 $cm^3$ d'eau, évapore le solvant et récupère le produit attendu.

Les détails de synthèse et les caractéristiques analytiques figurent au tableau II.

Exemples 15 à 20

Synthèse de dihalogénures de départ de formule (II)

On ajoute sous bonne agitation 0,35 mole de trichlorure d'aluminium à un mélange de 0,31 mole de chlorure d'acide carboxylique ou sulfonique et de 1,5 mole de halobenzène à température ambiante. On

porte le milieu à 100° C pendant 6 heures, puis on le verse sur un mélange de glace et d'acide chlorhydrique. Après décantation, on lave la phase organique avec une solution de carbonate de sodium puis avec de l'eau. On sèche sur sulfate de magnésium, évapore le solvant et purifie par recristallisation dans le n-hexane.

Les détails des synthèses et les caractéristiques analytiques figurent au tableau III.

Exemples 21 à 29

Synthèse de polymères α-w-diacétyléniques

On introduit dans un réacteur muni d'un séparateur de Dean et Starck, 0,55 mole de diphénol, 0,50 mole de dihalogénure, 90 g de carbonate de potassium, 900 $cm^3$ de n-méthylpyrrolidone et 300 $cm^3$ de toluène. On porte le milieu à 160° C pendant 2 heures puis 2 heures à 180° C. Après distillation du toluène, on ajoute alors 0,10 mole de composé acétylénique (agent de fonctionnalisation) et 150 $cm^3$ de toluène. Après 2 heures à 140° C, on refroidit, filtre et précipite le milieu dans 7 à 10 fois son volume de méthanol. On filtre, lave à l'eau, puis sèche à 100° C le précipité obtenu.

Les détails de synthèse et les caractéristiques analytiques figurent au tableau IV.

Exemples 30 à 36

Mise en oeuvre des polymères acétyléniques

On introduit 10 g de polymère acétylénique dans un moule métallique de 50 x 50 mm. On place le moule avec son poinçon entre les plateaux d'une presse et soumet à un cycle thermique défini par une température de 250 à 270° C, une pression de 15 à 30 bars et une durée de 10 minutes. Après démoulage, on effectue sur les éprouvettes des mesures de température de transition vitreuse, de K1C (Norme française 03-180) et de tenue aux solvants.

Les détails figurent au tableau V.

Exemple 37

Réalisation d'un composite

Avec le polymère préparé selon l'exemple 30, on a préparé un composite par imprégnation d'un tissu de carbone (T300-340 $g/m^2$) au moyen d'une solution dudit polymère dans du dichlorométhane. Après évaporation du solvant, 7 plis sont empilés dans un moule, placés entre les plateaux d'une presse. On a effectué la mise en forme à 250° C sous 50 à 100 bars durant 20 minutes. Le composite présente une allure saine, exempte de porosités ou de microfissurations. Le taux de fibre est de 71 % en poids et 63 % en volume. Les contraintes de résistance au cisaillement et en flexion sont respectivement de 61 et 719 MPa.

TABLEAU I

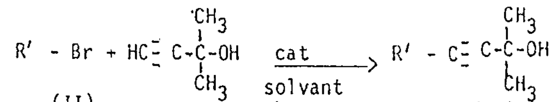


(II) (III) (IA)

| Exemple N° | R' - Br : R' - | R' - Br : Nombre (mole) | Méthyl-butyne-ol (mole) | Catalyseurs $Pd(\emptyset_3P)_2Cl_2$ $\emptyset_3P$ CuI (mg) | Base/ solvant | Conditions | Rendement % | Température de fusion °C |
|---|---|---|---|---|---|---|---|---|
| 1 | 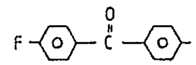 | 0,1075 | 0,160 | 107 215 215 | $Et_3N$ 800 cm³ | 90°C 6 H | 100 | 105 |
| 2 | 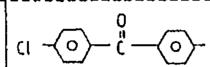 | 0,05 | 0,76 | 5 10 10 | $Et_3N$ | 90°C 8 H | 100 | 137 |
| 3 | 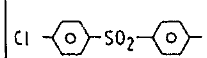 | 0,095 | 0,152 | 48 95 95 | $Et_3N$ 300 cm³ | 90°C 10 H | 100 | 98 |
| 4 | 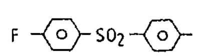 | 0,100 | 0,150 | 100 200 200 | $Et_3N$ 500 cm³ | 90°C 10 H | 100 | - |
| 5 | 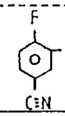 | 0,075 | 0,1125 | 37,5 75 75 | $Et_3N$ 100 cm³ | 90°C 7 H | 100 | 62 |
| 6 | 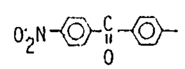 | 0,0345 | 0,0448 | 17 34 34 | $Et_3N$ 50 cm³ | 90°C 8 H | 94 | 135 |
| 7 | 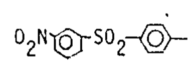 | 0,050 | 0,065 | 25 50 50 | $Et_3N$ 60 cm³ | 90°C 6 H | 94 | - |

TABLEAU II

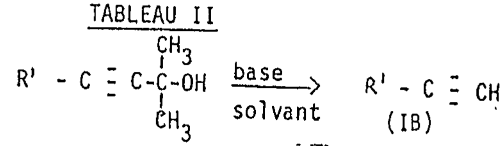


| Exemple N° | 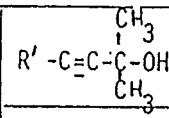 R′ - | Nombre (mole) | Base | Solvant | Conditions | Rendement % | Température de fusion °C |
|---|---|---|---|---|---|---|---|
| 8 | 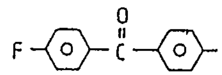 | $17\ 10^{-3}$ | NaOH 2,5 g | Ø-Cl 150 cm³ | 110°C 2 H | 90 | 93 |
| 9 | 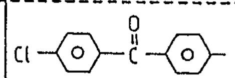 | $17\ 10^{-3}$ | NaOH 1 g | Ø-Cl 200 cm³ | 110°C 2 H | 90 | - |
| 10 | 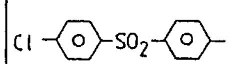 | $13,5\ 10^{-3}$ | NaOH 0,84 g | Ø-Cl 200 cm³ | 110°C 2 H | 92 | 146 |
| 11 | 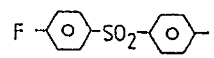 | $10\ 10^{-3}$ | NaOH 1,g | Ø-Cl 150 cm³ | 110°C 2 H | - | - |
| 12 | 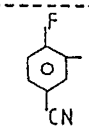 | $49\ 10^{-3}$ | NaOH 3 g | Ø-Cl 300 cm³ | 110°C 2 H | 63 | 70 |
| 13 | 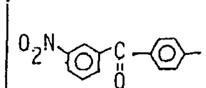 | 0,108 | NaH 3,4g | Ø-$CH_3$ 400 cm³ | 110°C 10 H | 63 | 126 |
| 14 | 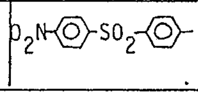 | 0,150 | NaH 3,6g | Ø-$CH_3$ 500 cm³ | 110°C 10 H | 73 | 132 |

TABLEAU III

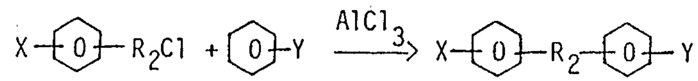

(IIA)

| Exemple N° | 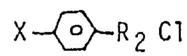 | | | 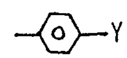 | | $AlCl_3$ (nb de moles) | Conditions | Rendement % | Température de fusion °C |
|---|---|---|---|---|---|---|---|---|---|
| | X | $R_1$ | Nombre (mole) | Y | Nombre (mole) | | | | |
| 15 | -F | $-\overset{O}{\overset{\|}{C}}-$ | .0,3155 | -Br | 1,30 | 0,34 | 100°C/16 H | 60 | 108 |
| 16 | -Cl | $-\overset{O}{\overset{\|}{C}}-$ | 0,34 | -Br | 1,70 | 0,37 | 100°C/10 H | 56 | 150 |
| 17 | -Cl | $-SO_2$ | 0,20 | -Br | 2,37 | 0,30 | 100°C/10 H | 57 | 159 |
| 18 | -F | $-SO_2$ | 0,10 | -Br | 1,10 | 0,15 | 100°C/10 H | Non mesuré | 115 |
| 19 | $-NO_2$ | $-\overset{O}{\overset{\|}{C}}-$ | 0,10 | -Br | 0,60 | 0,12 | 100°C/6 H | 72 | 122 |
| 20 | m $-NO_2$ | $-SO_2-$ | 0,12 | -Br | 0,80 | 0,15 | 100°C/6 H | 42 | 119 |

## TABLEAU IV

$$HO - \boxed{R_3} - OH + X - \boxed{R_4} - X + X - R' - C \equiv CH \ (I) \longrightarrow HC \equiv C - R' - O - \boxed{R_3} - \left[ O - \boxed{R_4} - O - \boxed{R_3} \right]_n - O - R' - C \equiv CH$$

| Exemple N° | $HO - R_3 - OH$ | $X - R_4 - X$ | $-R' - C \equiv CH$ | Rendement % | Température de transition vitreuse °C | GPC (2) $\bar{M}n$ | $\bar{M}w$ | $\bar{M}w/\bar{M}n$ |
|---|---|---|---|---|---|---|---|---|
| 21 | 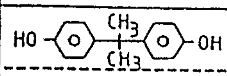 | 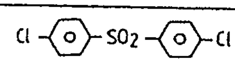 | 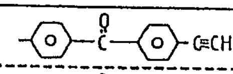 | 95 | 181 | 5170 | 7770 | 1,50 |
| 22 | " | " | 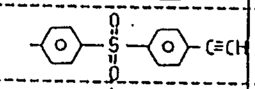 | 95 | 180 | | | |
| 23 | " | " | 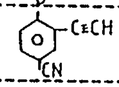 | 97 | 180 | | | |
| 24 | " | 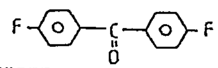 | 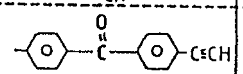 | 95 | 152 | 4930 | 7850 | 1,59 |
| 25 | " | 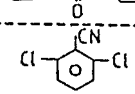 | " | 76 | 171 | 4740 | 6630 | 1,40 |
| 26 | 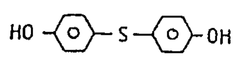 | " | " | 89 | 150 | 3540 | 5410 | 1,52 |
| 27 | " | 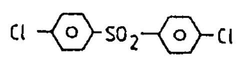 | " | 90 | 153 | 3800 | 5570 | 1,46 |
| 28 | 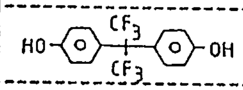 | " | " | 92 | 185 | 4140 | 5320 | 1,28 |
| 29 | " | 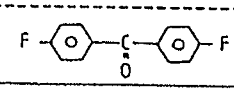 | " | 88 | 160 | 4730 | 7390 | 1,56 |

1) Température de transition vitreuse du polymère non réticulé mesurée par DSC à 20°C/mn

2) Masses molaires mesurées pàr GPC (solvant chloroforme - étalonnage interne polysulfone)

TABLEAU V

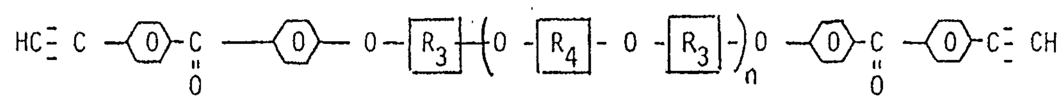

| Exemple N° | -R3- | -R4- | Mn | Mise en oeuvre T° °C | P bar | temps | Température de transition vitreuse °C | Température début perte de poids °C | Propriétés mécaniques (3) E MPa | K1C MPa.m^0,5 | Solubilité dans le chloroforme * |
|---|---|---|---|---|---|---|---|---|---|---|---|
| 30 | 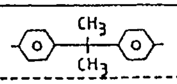 | 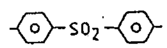 | 5170 | 250 | 20 | 10 min | 192 | 480 | 2310 | 1,80 | 6,0 |
| 31 | " | 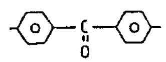 | 4930 | 270 | 13 | 10 min | 167 | 475 | 1690 | 3,90 | 0,7 |
| 32 | " | 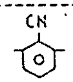 | 4740 | 270 | 15 | 10 min | 188 | 435 | 1770 | 1,90 | 0,0 |
| 33 | 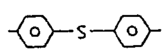 | " | 3540 | 270 | 20 | 10 min | 158 | 405 | 1820 | 0,85 | 13,0 |
| 34 | " | 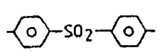 | 3800 | 270 | 20 | 10 min | 170 | 445 | 1660 | 0,85 | 6,0 |
| 35 | 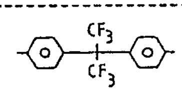 | " | 4140 | 270 | 30 | 10 min | 211 | 430 | 1960 | 1,60 | 4,10 |
| 36 | " | 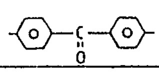 | 4730 | 270 | 10 | 10 min | 187 | 470 | 2060 | 3,00 | 0,0 |

1) mesurée par calorimétrie différentielle à 20°C/minute
2) analyse thermogravimétrique à 10°C/minute
3) E = module de flexion - $K_1C$ = facteur d'intensité de contrainte critique (norme française 03-180)
4) 48 H à température ambiante

Revendications

1. Dérivés acétyléniques, caractérisés en ce qu'ils répondent à la formule générale (I) :

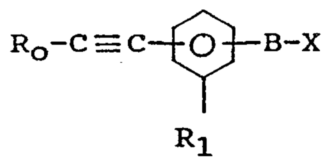

(I)

dans laquelle $R_0$ représente un atome d'hydrogène ou un radical isopropanol ou triméthylsilyl, $R_1$ représente un atome d'hydrogène ou un radical cyano ou nitro, X représente un atome de chlore ou de fluor ou un radical nitro et B représente une liaison carbone-halogène ou un radical :

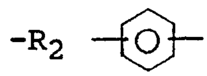

dans lequel $R_2$ représente un groupement carbonyle ou sulfone, étant entendu que si B représente un radical

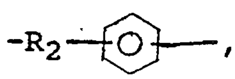

$R_1$ représente un atome d'hydrogène et si B représente une liaison carbone-halogène, $R_1$ ne représente pas un atome d'hydrogène étant entendu que lorsque B représente un radical :

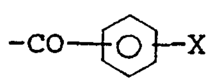

et $R_0$ représente un atome d'hydrogène, X ne représente pas un atome de fluor ou de chlore.

2. Dérivés acétyléniques répondant à la formule générale (I) de la revendication 1, caractérisés en ce que $R_0$ représente un atome d'hydrogène.

3. Dérivés acétyléniques répondant à la formule générale (I) de la revendication 1 ou 2, caractérisés en ce que B représente un radical

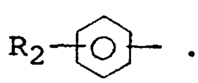

4. Dérivés acétyléniques répondant à la formule générale (I) de l'une quelconque des revendications 1 à 3, caractérisés en ce que $R_2$ représente un groupement carbonyle.

5. Dérivés acétyléniques répondant à la formule générale (I) de l'une quelconque des revendications 1 à 4, caractérisés en ce que X représente un atome de fluor.

6. Procédé de préparation des dérivés de formule (I) tels que définis à la revendication 1, ainsi que de ceux pour lesquels B représente un radical

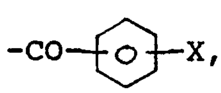

$R_0$ représente l'hydrogène et X représente le fluor ou le chlore, caractérisé en ce que l'on fait réagir un dérivé de formule (II) :

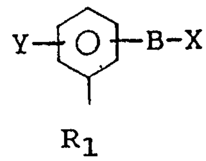

(II)

dans laquelle Y représente un atome de brome ou d'iode et B, X et $R_1$ ont la signification déjà indiquée, avec un dérivé de l'acétylène de formule (III) :

$R_0-C\equiv CH$ (III)

dans laquelle $R_0$ a la signification déjà indiquée, à l'exception de l'hydrogène, pour obtenir un dérivé de formule (IA) :

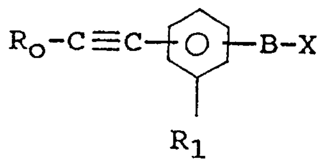

(IA)

dans laquelle $R_0$, $R_1$, B et X ont la signification déjà indiquée que soit l'on isole, soit l'on clive pour obtenir un dérivé de formule (IB) dans laquelle $R_1$, B et X ont la signification déjà indiquée :

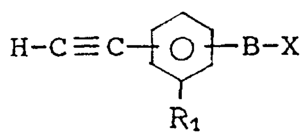

(IB)

7. Procédé selon la revendication 6, caractérisé en ce que :
- la réaction du dérivé de formule (I) avec le dérivé acétylénique de formule (III) est réalisée en présence d'une base, de préférence une amine tertiaire ;
- la réaction du dérivé de formule (II) avec le dérivé acétylénique de formule (III) est réalisée en présence d'un catalyseur à base de palladium, et de préférence le système catalytique à base de bis-(triphénylphosphine)-dichloropalladium, de triphénylphosphine et d'iodure de cuivre ;
- le clivage du dérivé de formule (IA) est réalisé à l'aide d'un hydroxyde alcalin.

8. Les polymères $\alpha$-w-acétyléniques, caractérisés en ce que la fonction acétylénique est conférée par un groupement

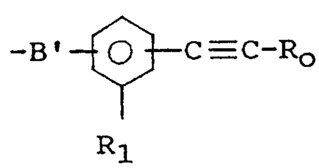

terminal dans lequel $R_0$ et $R_1$ ont la signification indiquée et $B'$ représente une liaison C-O ou un radical

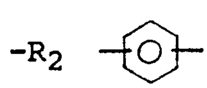

relié audit polymère par une fonction éther, $R_2$ ayant la signification déjà indiquée.

9. Application des dérivés de formule (I) tels que définis à la revendication 1, ainsi que de ceux pour lesquels B représente un radical

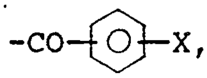

$R_0$ représente l'hydrogène et X représente le fluor ou le chlore, à l'éthynilation de dérivés polymériques ou oligomériques hydroxylés.

10. Produits conformés, caractérisés en ce qu'ils comprennent un polymère ou copolymère tel que défini dans l'une quelconque des revendications 1 à 5.

Office européen des brevets

# RAPPORT DE RECHERCHE EUROPEENNE

Numéro de la demande

**EP 90 40 1898**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-A-3 421 949 (UNION CARBIDE)<br>* revendication 1 *<br>– – – | 8,10 | C 07 C 49/835<br>C 07 C 317/22<br>C 07 C 255/53<br>C 07 C 49/813<br>C 07 C 317/14<br>C 07 C 255/50<br>C 08 G 75/23<br>C 08 G 65/48<br>C 07 C 205/57 //<br>C 07 C 317/32 |
| A<br>X | MAKROMOLEKULARE CHEMIE vol. 185, no. 9, septembre 1984, pages 1867-1880, Heidelberg, DE; V. PERCEC et al.: "Functional polymers and seqential copolymers by phase transfer catalysis, 2. Synthesis and characterization of aromatic poly(ether-sulfone)s containing vinylbenzyl and ethylbenzyl chain ends"<br>* page 1868, schéma 1, composé 4 *<br>– – – | 1<br>8,10 | |
| A | idem<br>– – – | 1 | |
| A | FR-A-2 371 195 (ROUSSEL UCLAF)<br>* revendications 1,3,4 *<br>– – – | 1-5 | |
| A | EP-A-0 311 349 (ICI)<br>* page 4, lignes 4-10 *<br>– – – | 1,8 | |
| D,A | JOURNAL OF POLYMER SCIENCE. POLYMER CHEMISTRY EDITION vol. 22, no. 11, part 1, novembre 1984, pages 3011-3025, New York, US; S.J. HAVENS: "Ethynyl-Terminated Polyarylates: Synthesis and Characterization"<br>* le document en entier *<br>– – – | 1 | **DOMAINES TECHNIQUES RECHERCHES (Int. Cl.5)**<br>C 07 C 49/00<br>C 07 C 317/00<br>C 07 C 255/00<br>C 08 G 85/00<br>C 08 G 65/00 |
| A | US-A-4 540 833 (WALLACE)<br>* revendications 1,4,5 *<br>– – – | 1 | |
| A | EP-A-0 052 599 (HOFFMANN-LA-ROCHE)<br>* revendication 1 *<br>– – –<br>–/– | 1 | |

Le présent rapport de recherche a été établi pour toutes les revendications

| Lieu de la recherche | Date d'achèvement de la recherche | Examinateur |
|---|---|---|
| Berlin | 18 octobre 90 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

&: membre de la même famille, document correspondant

Office européen des brevets

# RAPPORT DE RECHERCHE EUROPEENNE

Numéro de la demande

EP 90 40 1898

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 108 926 (ARNOLD)<br>* colonne 1, lignes 55-64; revendication 1 *<br>– – – | 1 | |
| A | DE-A-2 334 425 (W.H. RORER)<br>* revendication 1 *<br>– – – | 1 | |
| A | DD-A-1 235 96 (LILLY)<br>* revendication 1 *<br>– – – – – | 1 | |
| | | | DOMAINES TECHNIQUES RECHERCHES (Int. Cl.5) |

Le présent rapport de recherche a été établi pour toutes les revendications

| Lieu de la recherche | Date d'achèvement de la recherche | Examinateur |
|---|---|---|
| Berlin | 18 octobre 90 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant